(19) Europäisches Patentamt
European Patent Office
Office européen des brevets

(11) Veröffentlichungsnummer : **0 031 042**
**B1**

(12) # EUROPÄISCHE PATENTSCHRIFT

(45) Veröffentlichungstag der Patentschrift : **26.01.83**

(21) Anmeldenummer : **80107341.2**

(22) Anmeldetag : **25.11.80**

(51) Int. Cl.³ : **C 07 D471/04**, C 07 D487/04, A 01 N 25/32 // (C07D471/04, 233/00, 239/00, 243/00, 221/00),(C07D487/04, 233/00, 239/00, 243/00, 209/00)

(54) **N-Dichloroacethyldiazabicycloderivate, Verfahren zu ihrer Herstellung, herbizide Mittel, die Acetanilide als herbizide Wirkstoffe und diese N-Dichloroacetyldiazaantagonitische Mittel enthalten, sowie ihre Verwendung zur Bekämpfung unerwünschten Pflanzenwuchses.**

(30) Priorität : **03.12.79 DE 2948535**

(43) Veröffentlichungstag der Anmeldung : **01.07.81 Patentblatt 81/26**

(45) Bekanntmachung des Hinweises auf die Patenterteilung : **26.01.83 Patentblatt 83/04**

(84) Benannte Vertragsstaaten : **AT BE CH DE FR GB IT LI NL SE**

(56) Entgegenhaltungen :
**DE A 1 802 468**
**DE A 2 218 097**
**DE A 2 245 471**

(73) Patentinhaber : **BASF Aktiengesellschaft**
**Carl-Bosch-Strasse 38**
**D-6700 Ludwigshafen (DE)**

(72) Erfinder : **Rohr, Wolfgang, Dr.**
**In der Dreispitz 13**
**D-6706 Wachenheim (DE)**
Erfinder : **Hansen, Hanspeter, Dr.**
**Thorwaldsenstrasse 5**
**D-6700 Ludwigshafen (DE)**
Erfinder : **Plath, Peter, Dr.**
**Berner Weg 24**
**D-6700 Ludwigshafen (DE)**
Erfinder : **Wuerzer, Bruno, Dr.**
**Ruedigerstrasse 13**
**D-6701 Otterstadt (DE)**

**EP 0 031 042 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

N-Dichloracetyldiazabicycloderivate, Verfahren zu ihrer Herstellung, herbizide Mittel, die Acetanilide als herbizide Wirkstoffe und diese N-Dichloracetyldiazabicycloderivate als antagonistische Mittel enthalten, sowie ihre Verwendung zur Bekämpfung unerwünschten Pflanzenwuchses

Die vorliegende Erfindung betrifft neue Dichloracetamide, ein Verfahren zu ihrer Herstellung, herbizide Mittel, die Acetanilide als herbizide Wirkstoffe und diese Dichloracetamide als antagonistische Mittel enthalten, sowie Verfahren zur selektiven Bekämpfung von unerwünschtem Pflanzenwuchs mit diesen herbiziden Mitteln.

Acetanilide der Formel II

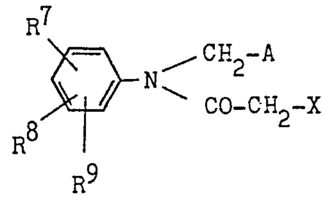

(II)

in der

$R^7$ Wasserstoff, einen unverzweigten oder verzweigten Alkyl- oder Alkoxyrest mit bis zu 5 Kohlenstoffatomen,

$R^8$ Wasserstoff, Halogen, einen unverzweigten oder verzweigten Alkyl- oder Alkoxyrest mit bis zu 5 Kohlenstoffatomen,

$R^9$ Wasserstoff, Halogen, einen unverzweigten oder verzweigten Alkyl- oder Alkoxyrest mit bis zu 5 Kohlenstoffatomen,

$R^7$ zusammen mit $R^8$ eine orthoständig verknüpfte, gegebenenfalls durch unverzweigte oder verzweigte Alkylgruppen mit bis zu 4 Kohlenstoffatomen substituierte Alkylenketten mit bis zu 6 Kohlenstoffatomen,

X Chlor oder Brom und

A einen Alkoxy- oder Alkoxyalkylrest mit bis zu 4 Kohlenstoffatomen oder ein über ein Ringstickstoffatom gebundenes Azol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy oder Alkoxycarbonyl mit bis zu 4 Kohlenstoffatomen in der Alkoxygruppe substituiert sein kann, wobei A auch für Salze der 2 oder 3 Stickstoffatome enthaltenden Azole stehen kann, bedeuten, sind herbizid ausgezeichnet wirksam, führen aber bei Anwendung in Kulturen, wie Mais, oder auch in anderen Kulturen aus der Familie der Gramineen zu Schädigungen der Nutzpflanzen (DE-A 26 48 008, DE-A 27 44 396).

Aufgabe der Erfindung war es deshalb, antagonistische Mittel zu finden, die diese Unverträglichkeit der herbiziden Acetanilide gegenüber bestimmten Kulturpflanzen kompensieren.

Herbizide Mittel, die neben herbiziden Wirkstoffen Dichloracetamide als antagonistisch wirkende Verbindungen enthalten, sind aus der DE-A 22 18 097 und der DE-A 22 45 471 bekannt. Die in der DE-A 22 18 097 beschriebenen Dichloracetamide werden vorwiegend zur Antagonisierung unerwünschter Kulturpflanzenschädigungen durch Thiolcarbamate verwendet, während aus der DE-A 22 45 471 auch herbizide Mittel bekannt sind, die Dichloracetamide und Chloracetanilide, beispielsweise 2-Chlor-2',6'-diäthyl-N-butoxymethylacetanilid oder 2-Chlor-2',6'-diäthyl-N-methoxymethylacetanilid, enthalten.

Es wurde gefunden, daß Dichloracetamide der Formel (I)

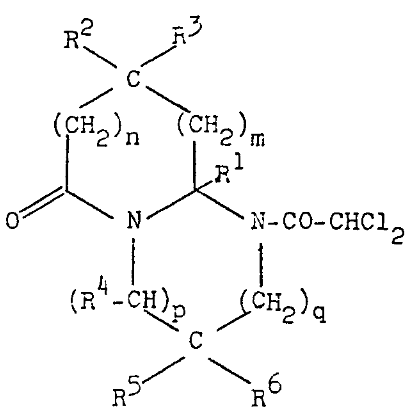

(I)

in der

$R^1$ für Wasserstoff, Methyl oder Ethyl,

$R^2$ und $R^3$ gleich oder verschieden sind und für Wasserstoff, Methyl oder Methoxy,

$R^4$ für Wasserstoff oder Methyl und

$R^5$ und $R^6$ gleich oder verschieden sind und für Wasserstoff oder Methyl stehen und

m 0 oder 1, n 1 oder 2, p 0, 1 oder 2 und q 0, 1 oder 2 bedeuten, sich ausgezeichnet zur Steigerung der Kulturpflanzenverträglichkeit von herbiziden Acetaniliden der Formel II eignen. Herbizide Mittel, die ein Acetanilid der Formel II und ein Dichloracetamid der Formel I enthalten, können sowohl in Mais als auch in Getreidekulturen eingesetzt werden. Dabei bleibt die gute herbizide Wirkung der Acetanilide erhalten, während Schädigungen an den Nutzpflanzen unterbunden werden.

Als antagonistische Mittel kommen Dichloracetamide von Diazabicycloalkanen der Formel I in Betracht, bei denen $R^1$ für Wasserstoff, Methyl oder Ethyl, $R^2$ und $R^3$ gleich oder verschieden sind und für Wasserstoff, Methyl oder Methoxy, $R^4$ für Wasserstoff oder Methyl und $R^5$ und $R^6$ gleich oder verschieden sind und für Wasserstoff oder Methyl stehen und m 0 oder 1, n 1 oder 2, p 0, 1 oder 2 und q 0, 1 oder 2 bedeuten. Bevorzugt stehen $R^1$ für Methyl, $R^2$, $R^3$, $R^4$, $R^5$ und $R^6$ für Wasserstoff, während m 0 und n, p und q 1 bedeuten.

Beispiele für Antidots der Formel I sind 4-Dichloracetyl-8-oxo-1,4-diaza-bicyclo[3.3.0]octan, 4-Dichloracetyl-5-methyl-8-oxo-1,4-diazabicyclo[3.3.0]octan, 4-Dichlor-5-ethyl-8-oxo-1,4-diazabicyclo-[3.3.0]octan, 5-Dichloracetyl-9-oxo-1,5-diazabicyclo[4.3.0]nonan, 5-Dichloracetyl-6-methyl-9-oxo-1,5-diazabicyclo[4.3.0]nonan, 5-Dichloracetyl-6-ethyl-9-oxo-1,5-diazabicyclo[4.3.0]nonan, 6-Dichloracetyl-7-methyl-10-oxo-1,6-diazabicyclo[5.3.0]decan, 7-Dichloracetyl-2-oxo-1,7-diazabicyclo[4.3.0]nonan, 7-Dichloracetyl-6-methyl-2-oxo-1,7-diazabicyclo[4.3.0]nonan, 7-Dichloracetyl-6-methyl-2-oxo-1,7-diazabicyclo[4.4.0]decan, 5-Dichloracetyl-3,3,6-trimethyl-9-oxo-1,5-diazabicyclo[4.3.0]nonan, 5-Dichloracetyl-4,4,6-trimethyl-9-oxo-1,5-diazabicyclo[4.3.0]nonan, 5-Dichloracetyl-4,4,6-trimethyl-10-oxo-1,5-diazabicyclo[4.4.0]decan, 5-Dichloracetyl-3,3,6-trimethyl-10-oxo-1,5-diazabicyclo[4.4.0]decan, 7-Dichloracetyl-5-methoxy-6-methyl-2-oxo-1,7-diazabicyclo[4.3.0]nonan, 5-Dichloracetyl-3,3-dimethyl-9-oxo-1,5-diazabicyclo[4.3.0]nonan, 5-Dichloracetyl-6-methyl-7-methoxy-10-oxo-1,5-diazabicyclo[4.4.0]decan. Bevorzugte antagonistische Mittel sind 4-Dichloracetyl-5-methyl-8-oxo-1,4-diazabicyclo-[3.3.0]octan, 5-Dichloracetyl-6-methyl-9-oxo-1,5-diazabicyclo-[4.3.0]nonan und 5-Dichloracetyl-3,3,6-trimethyl-9-oxo-1,5-diazabicyclo[4.3.0]nonan.

Bevorzugte Verbindungen der Formel I sind beispielsweise 5-Dichloracetyl-6-methyl-9-oxo-1,5-diazabicyclo-[4.3.0]nonan, 5-Dichloracetyl-3,3,6-trimethyl-9-oxo-1,5-diazabicyclo[4.3.0]nonan und 4-Dichloracetyl-5-methyl-8-oxo-1,4-diazabicyclo-[3.3.0]octan.

Dichloracetamide der Formel I können in an sich bekannter Weise durch Umsetzung von Diazabicycloalkanen der Formel III

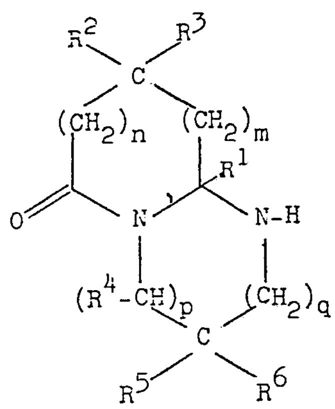

(III)

in der $R^1$, $R^2$, $R^3$, $R^4$, $R^5$, $R^6$ m, n, p und q die oben genannten Bedeutungen haben, mit Dichloracetylchlorid erhalten werden. Die Umsetzung wird in einem Lösungs- oder Verdünnungsmittel in Gegenwart eines chlorwasserstoffbindenden Mittels bei einer Temperatur im Bereich zwischen − 10 und + 20 °C durchgeführt. Dabei werden die Ausgangsstoffe im allgemeinen in äquimolarem Verhältnis eingesetzt. Es ist aber auch möglich, mit einem Überschuß an Dichloracetylchlorid zu arbeiten.

Als Verdünnungs- oder Lösungsmittel kommen gegebenenfalls halogenierte Kohlenwasserstoffe, wie Toluol, Xylole, Chlorbenzol, Dichlormethan, Ethylenchlorid, Ether, wie Diethylether, Methyl-tert.-butylether, Tetrahydrofuran, 1,4-Dioxan, oder Nitrile, wie Acetonitril, in Betracht.

Geeignete chlorwasserstoffbindende Mittel sind Alkalimetallcarbonate, Alkalimetallhydrogencarbonate, Alkalimetallhydroxide, Trialkylamine, N,N-Dialkylaniline und Pyridinbasen.

Die erfindungsgemäßen Dichloracetamide können auch durch Umsetzung von Diazabicycloalkanen der Formel III mit Chloralhydrat in Gegenwart eines säurebindenden Mittels und katalytischen Mengen Cyanid, das z.B. in Form von Natriumcyanid oder Acetoncyanhydrin zugegeben wird, erhalten werden (DE-A 28 07 340).

Die bicyclischen Amine der Formel II sind zum Teil aus der DE-A 02 468 bekannt. Sie lassen sich nach dem dort beschriebenen Herstellungsverfahren durch Umsetzung von γ-Oxo- bzw. δ-Oxo-carbonsäuren oder deren Estern mit α,ω-Alkylendiaminen erhalten. So kann z.B. 6-Methyl-9-oxo-1,5-diazabicyclo[4.3.0]nonan aus Lävulinsäureethylester und Propylendiamin hergestellt werden.

Das folgende Beispiel erläutert die Herstellung der neuen Dichloracetamide der Formel I.

Beispiel

Zu einer Lösung von 15,4 g (0,1 Mol) 6-Methyl-9-oxo-1,5-diazabicyclo[4.3.0]nonan in 100 ml Toluol gibt man 10,5 g (0,105 Mol) Triethylamin. Anschließend kühlt man auf − 10 °C ab und tropft bei − 10 bis − 5 °C 14,8 g (0,1 Mol) Dichloracetylchlorid zu. Nach 4-stündigem Nachrühren wird vom ausgefallenen Hydrochlorid des Triethylamins abgetrennt. Aus dem Filtrat scheiden sich nach dem Abdampfen des Toluols Kristalle ab, die aus Toluol/Aceton = 1 : 3 umkristallisiert werden können. Man erhält 17,7 g 5-Dichloracetyl-6-methyl-9-oxo-1,5-diazabicyclo[4.3.0]nonan vom Fp. 144 bis 147 °C.

Die tabellarisch aufgeführten Dichloracetamide können in analoger Weise erhalten werden. Bei unter Normalbedingungen flüssigen Verbindungen kann eine chromatographische Reinigung an Kieselgel (Laufmittel Essigester oder Toluol/Aceton (1 : 3)) erforderlich sein.

| Nr. | Formel | Fp. [°C] |
|---|---|---|
| 1 | 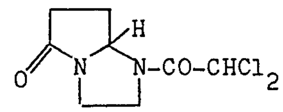 | 110 |
| 2 | 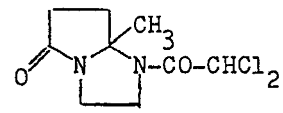 | 119-121 |
| 3 | 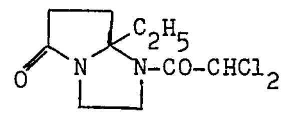 | Öl |
| 4 | 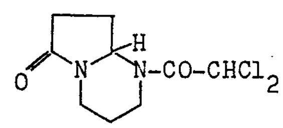 | 130 |
| 5 | 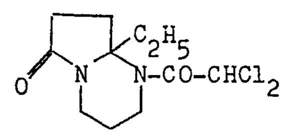 | Öl |
| 6 | 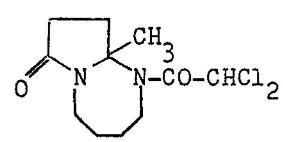 | |
| 7 | 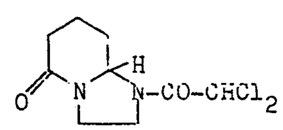 | |
| 8 | 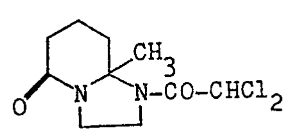 | 113-115 |
| 9 | 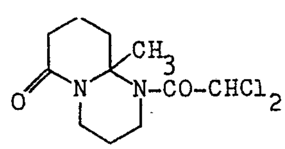 | 125 |

| | | |
|---|---|---|
| 10 | 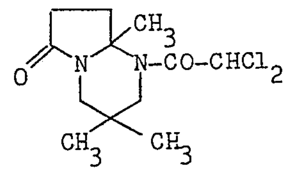 | 151-152 |
| 11 | 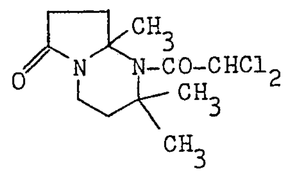 | 162-164 |
| 12 | 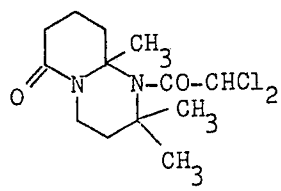 | |
| 13 | 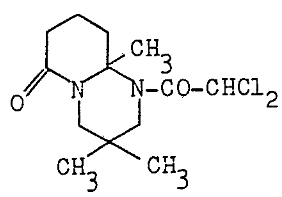 | |
| 14 | 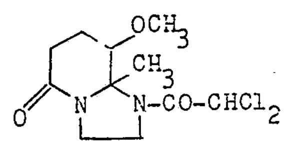 | Öl |
| 15 | 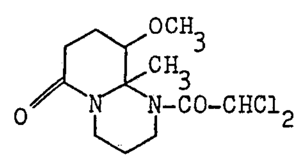 | 137-139 |
| 16 | 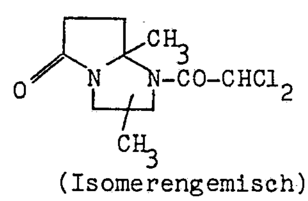<br>(Isomerengemisch) | |
| 17 | 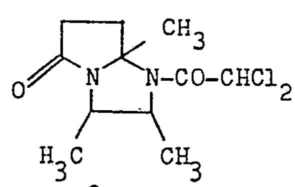 | 132-133 |
| 18 | 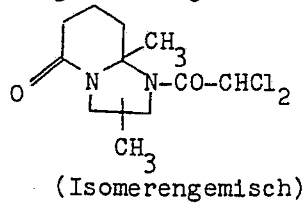<br>(Isomerengemisch) | |

19 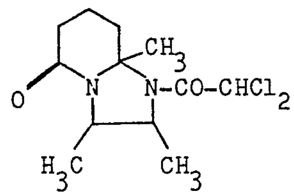

20 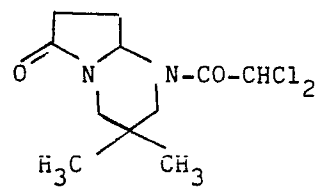 146

Acetanilide, deren Kulturpflanzenverträglichkeit durch die neuen Dichloracetamide der Formel I verbessert werden kann, sind solche der Formel II, bei denen $R^7$ für Wasserstoff, Alkyl bis zu 5 Kohlenstoffatomen wie Methyl, Ethyl, n-Propyl, i-Propyl, n-Butyl, sec.-Butyl, i-Butyl, tert.-Butyl, normale und verzweigte Pentylreste, Alkoxy mit bis zu 5 Kohlenstoffatomen, wie Methoxy, Ethoxy, Propoxy, Butoxy, Pentyloxy ;

$R^8$ und $R^9$ für Wasserstoff, Halogen, wie Fluor, Chlor, Brom oder Jod, Alkyl bis zu 5 Kohlenstoffatomen, wie Methyl, Ethyl, n-Propyl, i-Propyl, n-Butyl, sec.-Butyl, i-Butyl, tert.-Butyl, normale und verzweigte Pentylreste, Alkoxy mit bis zu 5 Kohlenstoffatomen, wie Methoxy, Ethoxy, Propoxy, Butoxy, Pentyloxy ;

$R^7$ zusammen mit $R^8$ für eine orthoständig verknüpfte, gegegebenenfalls durch Alkyl mit bis zu 4 Kohlenstoffatomen substituierte Alkylenkette mit bis zu 6 Kohlenstoffatomen, wie Ethylen, Trimethylen, Tetramethylen, 1-Methyl-trimethylen, 1,1-Dimethyl-trimethylen, 1,1-Dimethyl-tetramethylen ;

X für Chlor oder Brom, vorzugsweise Chlor, und

A für ein über ein Ring-Stickstoffatom gebundenes Azol, wie Pyrrol, Pyrazol, Imidazol, 1,2,4-Triazol, 1,2,3-Triazol, Tetrazol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy oder Alkoxycarbonyl mit bis zu 4 Kohlenstoffatomen in der Alkoxygruppe substituiert sein kann, oder für einen Alkoxy- oder Alkoxyalkylrest mit bis zu 4 Kohlenstoffatomen, wie Methoxy, Ethoxy, Methoxymethyl, 2-Methoxyethyl, stehen.

Beispiele für substituierte Azole für A sind 2,6-Dimethylpyrrol, Tetramethylpyrrol, 3(5)-Methylpyrazol, 4-Methylpyrazol, 3(5)-Ethylpyrazol, 4-Ethylpyrazol, 3(5)-Isopropylpyrazol, 4-Isopropylpyrazol, 3,5-Dimethylpyrazol, 3,4,5-Trimethylpyrazol, 3(5)-Phenylpyrazol, 4-Phenylpyrazol, 3,5-Diphenylpyrazol, 3(5)-Phenyl-5(3)-methylpyrazol, 3(5)-Chlorpyrazol, 4-Chlorpyrazol, 4-Brompyrazol, 3,5-Dimethyl-4-chlorpyrazol, 3,5-Dimethyl-4-brompyrazol, 4-Chlor-3(5)-methylpyrazol, 4-Methyl-3,5-dichlorpyrazol, 3(5)-Methyl-4,5(3)-dichlorpyrazol, 3(5)-Chlor-5(3)-methylpyrazol, 4-Methoxypyrazol, 3(5)-Methyl-5(3)-trifluormethylpyrazol, 3(5)-Methyl-5(3)-ethoxycarbonylpyrazol, 3(5)-Methyl-5(3)-methylthio-4-methoxy-carbonylpyrazol, 4-Cyanopyrazol, 4,5-Dichlor-imidazol, 2-Methyl-4,5-dichlorimidazol, 3(5)-Methyl-1,2,4-triazol, 3,5-Dimethyl-1,2,4-triazol, 3(5)-Chlor-1,2,4-triazol, 3,5-Dichlor-1,2,4-triazol, 4(5)-Methyl-1,2,3-triazol, 5-Methyltetrazol oder 5-Chlortetrazol.

Darüber hinaus kann der Rest A für den Fall, daß das Azol 2 oder 3 Stickstoffatome enthält, auch salzartig an eine der ublichen starken anorganischen oder organischen Säuren, wie Chlorwasserstoffsäure, Salpetersäure, Schwefelsäure, Trichloressigsäure, Methansulfonsäure, Perfluorhexansulfonsäure oder Dodecylbenzolsulfonsäure gebunden sein.

Bevorzugt sind Acetanilide der Formel II, die in 2- und 6-Stellung am Phenylring Methyl oder Ethyl und in 3-Stellung Wasserstoff oder Methyl tragen. X steht vorzugsweise für Chlor, während für A insbesondere ein Azolrest, wie Pyrazol, 4-Methylpyrazol, 4-Methoxypyrazol, 3-Methylpyrazol, 3,5-Dimethylpyrazol, 1,2,4-Triazol oder 4,5-Dichlorimidazol, Ethoxy oder Methoxymethyl in Betracht kommen.

Insbesondere enthalten die erfindungsgemäßen herbiziden Mittel folgende Acetanilide :

2-Chlor-2',6'-dimethyl-N-(pyrazol-1-yl-methyl)-acetanilid, 2-Chlor-2'-methyl-6'-ethyl-N-(pyrazol-1-yl-methyl)-acetanilid, 2-Chlor-2',6'-dimethyl-N-(4-methyl-pyrazol-1-yl-methyl)-acetanilid, 2-Chlor-2'-methyl-6'-ethyl-N-(4-methoxy-pyrazol-1-yl-methyl)-acetanilid, 2-Chlor-2',6'-dimethyl-N-(3,5-dimethylpyrazol-1-yl-methyl)-acetanilid, 2-Chlor-2'-methyl-6'-ethyl-N-(3-methyl-pyrazol-1-yl-methyl)-acetanilid, 2-Chlor-2',6'-dimethyl-N-(1,2,4-triazol-1-yl-methyl)-acetanilid, 2-Chlor-2',3',6'-trimethyl-N-(pyrazol-1-yl-methyl)-acetanilid, 2-Chlor-2'-methyl-6'-ethyl-N-(3,5-dimethyl-pyrazol-1-yl-methyl)-acetanilid, 2-Chlor-2',6'-diethyl-N-(3,5-dimethyl-pyrazol-1-yl-methyl)-acetanilid, 2-Chlor-2',3',6'-trimethyl-N-(3,5-dimethyl-pyrazol-1-yl-methyl)-acetanilid, 2-Chlor-2',6'-diethyl-N-(pyrazol-1-yl-methyl)-acetanilid, 2-Chlor-2',6'-dimethyl-N-(4,5-dichlorimidazol-1-yl-methyl)-acetanilid, 2-Chlor-2'-methyl-6'-ethyl-N-(4,5-dichlorimidazol-1-yl-methyl)-acetanilid, 2-Chlor-2',6'-diethyl-N-(4,5-dichlorimidazol-1-yl-methyl)-acetanilid, 2-Chlor-2'-methyl-6'-ethyl-N-(1,2,4-triazol-1-yl-methyl)-acetanilid, 2-Chlor-2',6'-diethyl-N-(1,2,4-triazol-1-yl-methyl)-acetanilid, 2-Chlor-2',3',6'-trimethyl-N-(1,2,4-triazol-1-yl-methyl)-acetanilid, 2-Chlor-2',6'-dimethyl-N-(2-methoxy-ethyl)-acetanilid und 2-Chlor-2'-methyl-6'-ethyl-N-ethoxymethyl-acetanilid.

Die Acetanilide der Formel II und ihre Herstellung sind Gegenstand der DE-A 26 48 008, der DE-A 27 44 396, der DE-A 23 05 495 und der US-A 3 547 620.

Herbizide Wirkstoffe und antagonistisch wirkende Verbindungen können gemeinsam oder getrennt vor oder nach der Saat in den Boden eingearbeitet werden. Bei den Acetaniliden ist das gebräuchlichste Anwendungsverfahren die Ausbringung auf die Erdoberfläche unmittelbar nach Ablage der Samen oder in der Zeit zwischen Einsaat und Auflaufen der jungen Pflanzen. Auch eine Behandlung während des Auflaufens ist möglich. In jedem Fall kann das antagonistisch wirkende Mittel gleichzeitig mit dem herbiziden Wirkstoff ausgebracht werden. Auch eine getrennte Ausbringung, wobei der Antagonist zuerst und anschließend der herbizide Wirkstoff oder umgekehrt auf das Feld gebracht werden, ist möglich, sofern zwischen Ausbringung beider Substanzen nicht soviel Zeit verstreicht, daß der herbizide Wirkstoff bereits Schaden an den Kulturpflanzen anrichtet. Wirkstoff und Antagonist können hierbei als Spritzmittel in suspendierbarer, emulgierbarer oder löslicher Form oder als Granulate getrennt oder gemeinsam formuliert vorliegen. Denkbar ist auch eine Behandlung der Kulturpflanzensamen mit dem Antagonisten vor der Aussaat. Der herbizide Wirkstoff wird dann allein in der üblichen Weise appliziert.

Für das gleiche herbizide Acetanilid werden unterschiedliche Mengen einer antagonistisch wirkenden Verbindung benötigt, wenn das Acetanilid in verschiedenen Kulturen eingesetzt wird. Die Mengenverhältnisse, in denen Acetanilid und Dichloracetamid eingesetzt werden, sind in breiten Bereichen variabel. Sie sind abhängig von der Struktur des Acetanilids, des Dichloracetamids und der jeweiligen Kultur. Geeignete Anteilsverhältnisse herbizider Wirkstoff : antagonistisch wirkender Verbindung liegen zwischen 1 : 2 bis 1 : 0,01, vorzugsweise 1 : 0,25 bis 1 : 0,05 Gewichtsteile.

Die neuen herbiziden Mittel können neben Acetanilid und Dichloracetamid weitere herbizide oder wachstumsregulierende Wirkstoffe anderer chemischer Struktur, beispielsweise 2-Chlor-4-ethylamino-6-isopropylamino-1,3,5-triazin, enthalten, wobei der antagonistische Effekt erhalten bleibt.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, ölige oder sonstige Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle, sowie Öle pflanzlichen oder tierischen Ursprungs, aliphatische, cyclische oder aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern) Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol, Nonylphenyl, Alkylphenolpolyglykolether, Tributylphenylpolyglykolether, Alkalarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubmittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Formulierungen enthalten zwischen 0,1 und 95 Gewichtsprozent an herbizidem Wirkstoff und Antidot, vorzugsweise zwischen 0,5 und 90 Gewichtsprozent. Die Aufwandmengen an herbizidem

Wirkstoff betragen 0,2 bis 5 kg/ha. Diese Menge an herbizidem Wirkstoff wird, gemeinsam oder getrennt, mit einer solchen Menge an Antidot ausgebracht, daß das Anteilsverhältnis herbizider Wirkstoff : antagonistischer Verbindung 1 : 2 bis 1 : 0,01, vorzugsweise 1 : 0,25 bis 1 : 0,05, Gewichtsteile beträgt.

Beispiele für Formulierungen sind :

I. 40 Gewichtsteile der Mischung aus vier Gewichtsteilen 2-Chlor-2',6'-dimethyl-N-(pyrazol-1-yl-methyl)-acetanilid und einem Gewichtsteil 5-Dichloracetyl-6-methyl-9-oxo-1,5-diazabicyclo[4.3.0]nonan werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

II. 3 Gewichtsteile der Mischung aus einem Gewichtsteil 2-Chlor-2',6'-dimethyl-N-(pyrazol-1-yl-methyl)-acetanilid und einem Gewichtsteil 4-Dichloracetyl-5-methyl-8-oxo-1,4-diazabicyclo[3.3.0]octan werden mit 97 Gewichtsteilen feinteiligem Kaolin innig vermischt. Man erhält auf diese Weise ein Stäubemittel, das 3 Gewichtsprozent des Wirkstoffs enthält.

III. 30 Gewichtsteile der Mischung aus einem Gewichtsteil 2-Chlor-2'-methyl-6'-ethyl-N-(1,2,4-triazol-1-yl-methyl)-acetanilid und zwei Gewichtsteilen 5-Dichloracetyl-3,3,6-trimethyl-9-oxo-1,5-diazabi-cyclo[4.3.0]nonan werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV. 20 Teile der Mischung aus 8 Gewichtsteilen 2-Chlor-2'-methyl-6'-ethyl-N-ethoxymethyl-acetanilid und einem Gewichtsteil 5-Dichloracetyl-4,4,6-trimethyl-9-oxo-1,5-diazabicyclo[4.3.0]nonan werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

V. 20 Gewichtsteile der Mischung aus 10 Gewichtsteilen 2-Chlor-2',6'-dimethyl-N-(2-methoxyethyl)-acetanilid und einem Gewichtsteil 4-Dichloracetyl-5-methyl-8-oxo-1,4-diazabicyclo[3.3.0]octan werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Der Einfluß verschiedener Vertreter der erfindungsgemäßen herbiziden Mittel auf das Wachstum von erwünschten und unerwünschten Pflanzen im Vergleich zu dem herbizider Mittel aus den gleichen herbiziden Wirkstoffen und einer antagonistisch wirkenden, bereits bekannten Verbindung chemisch ähnlicher Struktur wird durch die folgenden biologischen Beispiele belegt. Die Versuche zeigen, daß die Verträglichkeit der herbiziden Acetanilide durch kombinierte Anwendung mit den neuen Dichloracetamiden bei gleichbleibender herbizider Wirkung auch unter für die Herbizide und das Pflanzenwachstum schwierigen Bedingungen, wie z.B. bei sehr starkem Niederschlag, entscheidend verbessert wird.

Die Versuchsserien wurden im Gewächshaus und im Freiland durchgeführt.

## I. Gewächshausversuche

Plastikpikierkisten von 51 cm Länge, 32 cm Breite und 6 cm Höhe wurden mit lehmigem Sand von pH 6 und etwa 1,5 % Humus gefüllt. In dieses Substrat wurde Mais (Zea mays) in Reihen flach eingesät. Außerdem wurde Echinochloa crusgalli als unerwünschte Pflanze breitwürfig hinzugegeben. Der nicht sterilisierte Boden enthielt zusätzlich lebensfähige Unkrautsamen, welche zur Population unerwünschter Pflanzen beitrugen. Dadurch wurde ein mit Kulturpflanzen bestellter und mit Unkräutern verseuchter Acker simultiert.

Wirkstoffe und Antagonisten wurden sowohl einzeln auch auch in den beschriebenen Mischungen aufgebracht. Dazu verteilte man sie, emulgiert oder suspendiert in Wasser als Trägermedium, und spritzte sie mittels fein verteilender Düsen unmittelbar nach der Saat und vor Auflaufen der Testpflanzen auf die Erdoberfläche. In einigen Fällen wurden die Mittel auch vor Einsaat der Kulturpflanzen in den Boden eingemischt. Nach Einsaat und Behandlung wurden die Kisten beregnet und bis nach dem Auflaufen der Pflanzen mit durchsichtigen Plastikhauben abgedeckt. Durch diese Maßnahmen wurde ein gleichmäßiges Keimen und Anwachsen der Pflanzen garantiert. Die Aufstellung erfolgte in einem Temperaturbereich von durchschnittlich 18 bis 30 °C.

Die so angelegten Gewächshausversuche wurden beobachtet, bis der Mais 3 bis 5 Blätter entwickelt hatte. Nach diesem Stadium waren bei den herbiziden Mitteln keine Schädigungen mehr zu erwarten, was auch in den Freilandversuchen bestätigt wurde. Die Wirkung der Mittel wurde nach einer Skala von 0 bis 100 vorgenommen. Hierbei bedeutet 0 normaler Aufgang und Entwicklung der Pflanzen, bezogen auf die unbehandelte Kontrolle. 100 entsprach einem völligen Ausbleiben der Keimung bzw. Absterben der Pflanzen.

## II. Freilandversuche

Die Freilandversuche wurden auf Kleinparzellen auf Standorten mit lehmigem Sand und Lehm von

pH 5 bis 6 und 1 bis 1,5 % Humusgehalt angelegt. Die Vorauflaufbehandlungen erfolgten jeweils unmittelbar bis spätestens 3 Tage nach der Saat der Kulturpflanzen. Die Unkrautflora verschiedenster Artenzusammensetzung war natürlich. Allerdings wurden nur die dominierenden Vertreter in die folgenden Tabellen aufgenommen. Wirkstoffe und Antagonisten sowie deren Kombinationen wurden in Wasser als Träger- und Verteilermedium emulgiert oder suspendiert und mittels einer motorgetriebenen, auf einen Traktor montierten Parzellenspritze ausgebracht. Bei Mangel an natürlichen Niederschlägen wurde zusätzlich beregnet, um ein normales Auflaufen von Kultur und Unkräutern zu gewährleisten.

Bei einem Teil der Versuche wurde auf besonders harte Bedingungen für die herbiziden Mittel Wert gelegt und durch Niederschlagsüberschuß die Keim- und Wurzelzonen der Kulturpflanzen besonders der Einwirkung der herbiziden Acetanilide ausgesetzt, wobei die herbiziden Mittel selbst einer verstärkten Mobilität im Boden unterworfen waren. Die Bewertung der Mittelwirkung wurde ebenfalls nach der Skala von 0 bis 100 vorgenommen.

Im einzelnen zeigen die tabellarisch aufgeführten Versuchsergebnisse, daß die neuen antagonistisch wirkenden Dichloracetamide, für sich allein ausgebracht, keinen oder einen kaum sichtbaren Einfluß auf Keimung und Wachstum von Kulturpflanzen oder unerwünschten Pflanzen haben. Auch bei Aufwandmengen, welche beträchtlich über denjenigen liegen, welche für antagonistische Effekte erforderlich sind, sind sie herbizid unwirksam.

Die neuen Verbindungen reduzieren jedoch die Phytotoxizität der herbiziden Acetanilide der Formel II gegenüber Mais beachtlich und eliminieren sie zum Teil völlig, selbst unter außergewöhnlichen Einflüssen, wie z.B. starken Niederschlägen. Bei herbiziden Verbindungen, die gegenüber Kulturpflanzen weniger aggressiv sind, genügt bereits der Zusatz kleinerer Mengen antagonistisch wirkender Substanzen oder solcher mit geringerer antagonistischer Aktivität.

Tabelle 1 — Liste der Pflanzennamen

| Botanischer Name | Abkürzung in Tabellen | |
|---|---|---|
| Alopecurus myosuroides | Alopec. myos. | Ackerfuchsschwanz |
| Chenopodium album | Chenop. alb. | Weißer Gänsefuß |
| Echinochloa crus galli | Echinochl. crus-galli | Hühnerhirse |
| Zea mays | | Mais |

Tabelle 2 — Liste der in den biologischen Beispielen aufgeführten herbiziden Acetanilide

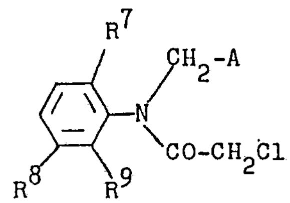

| Bezeichnung | A | $R^7$ | $R^8$ | $R^9$ |
|---|---|---|---|---|
| A | 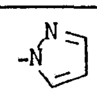 | $CH_3$ | H | $CH_3$ |
| B | 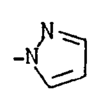 | $C_2H_5$ | H | $CH_3$ |
| C | 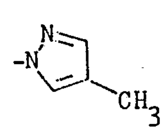 | $CH_3$ | H | $CH_3$ |

| | | | | |
|---|---|---|---|---|
| D | 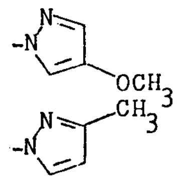 | $C_2H_5$ | H | $CH_3$ |
| E | | $C_2H_5$ | H | $CH_3$ |
| F | 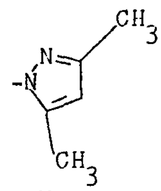 | $CH_3$ | H | $CH_3$ |
| G | 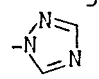 | $CH_3$ | H | $CH_3$ |
| H | 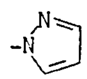 | $CH_3$ | $CH_3$ | $CH_3$ |
| I | $-O-C_2H_5$ | $CH_3$ | H | $C_2H_5$ |
| K | $-CH_2-O-CH_3$ | $CH_3$ | H | $CH_3$ |

Tabelle 3 — Liste der in den biologischen Beispielen aufgeführten antagonistisch wirkenden Verbindungen

| Nr. | Strukturformel |
|---|---|
| $A_1$ | 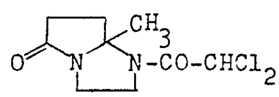 |
| $A_2$ | 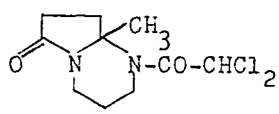 |
| $A_3$ | 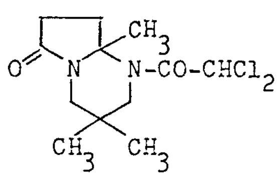 |
| $A_4$ | 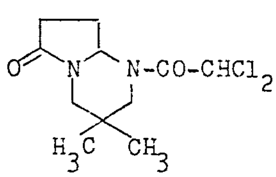 |
| $A_5$ | 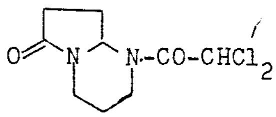 |

$A_6$

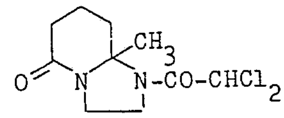

$A_7$

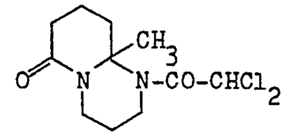

$A_8$

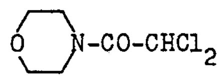

(DE-A 22 18 097)

$A_9$

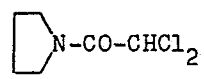

(DE-A 22 45 471)

(Siehe die Tabelle 4 Seite 12)

Tabelle 4 — Reduktion von Schäden an Mais durch 2-(Chlor-2',6'-dimethyl-N-(pyrazolyl-1-yl-methyl)-acetanilid bei Zugabe antagonistisch wirkender Dichloracetamide bei Vorauflaufanwendung im Gewächshaus

| Herbizider Wirkstoff | Antagonistisch wirkende Verbindung | Aufwandmenge (kg a.S./ha) | Testpflanzen und Schädigung (%) Kulturpflanze Zea mays | unerwünschte Pflanze Echinochloa crus-galli |
|---|---|---|---|---|
| | $A_1$ | 4,0 | 0 | 0 |
| | $A_2$ | 4,0 | 10 | 0 |
| | $A_3$ | 4,0 | 0 | 0 |
| A | - | 1,0 | 33 | 100 |
| | - | 2,0 | 85 | 100 |
| A | $A_1$ | 1,0 + 0,1 | 12 | 100 |
| | | 1,0 + 0,25 | 12 | 100 |
| | | 2,0 + 0,5 | 9 | 100 |
| A | $A_2$ | 1,0 + 0,1 | 8 | 100 |
| | | 1,0 + 0,25 | 0 | 99 |
| | | 2,0 + 0,5 | 10 | 100 |
| A | $A_3$ | 1,0 + 0,1 | 0 | 99 |
| | | 2,0 + 0,5 | 5 | 100 |
| A | $A_8$ (bekannt) | 2,0 + 0,5 | 36 | 99 |
| A | $A_9$ (bekannt) | 2,0 + 0,5 | 32 | 100 |

0 = normaler Aufgang, keine Schädigung
100 = Pflanzen nicht aufgelaufen oder abgestorben

Tabelle 5 — Antagonistische Wirkung eines Dichloracetamids zur Verhinderung von Schäden an Mais durch 2-Chlor-2',6'-dimethyl-N-(pyrazolyl-1-yl-methyl)-acetanilid unter schwierigen Bedingungen im Freiland

| Herbizider Wirkstoff | Antagonistisch wirkende Verbindung | Aufwandmenge (kg a.S./ha) | Testpflanzen und Schädigung (%) | | | |
|---|---|---|---|---|---|---|
| | | | Kulturpfl. Zea mays | Alopec. myos. | Echinochl. crus-galli | Chenop. album |
| A | - | 1,0 | 26 | 100 | 100 | 97 |
| | | 2,0 | 65 | 100 | 100 | 99 |
| A | $A_1$ | 1,0 + 0,125 | 3 | 100 | 100 | 99 |
| | | 1,0 + 0,25 | 2 | 100 | 100 | 99 |
| | | 2,0 + 0,25 | 8 | 100 | 100 | 100 |
| | | 2,0 + 0,5 | 4 | 100 | 100 | 100 |

0 = keine Schädigung
100 = Pflanzen nicht aufgelaufen oder abgestorben

Tabelle 6 — Reduktion von Schäden an Mais durch herbizide Halogenacetanilide bei Zugabe eines antagonistisch wirkenden Dichloracetamids bei Vorauflaufanwendung im Gewächshaus

| Herbizide Wirkstoffe | Antagonistisch wirkende Verbindung | Aufwandmenge (kg a.S./ha) | Testpflanzen und Schädigung (%) Kulturpflanze Zea mays | Testpflanzen und Schädigung (%) unerwünschte Pflanze Echinochloa crus-galli |
|---|---|---|---|---|
| B | | 1,0 | 30 | 98 |
| | | 2,0 | 80 | 100 |
| B | $A_3$ | 1,0 + 0,125 | 0 | 100 |
| | | 2,0 + 0,5 | 0 | 100 |
| | | 2,0 + 0,25 | 0 | 100 |
| C | | 1,5 | 30 | 98 |
| | | 2,5 | 40 | 98 |
| C | $A_3$ | 1,5 + 0,188 | 5 | 100 |
| | | 2,5 + 0,625 | 0 | 100 |
| D | | 1,5 | 20 | 90 |
| | | 2,5 | 35 | 90 |
| D | $A_3$ | 1,5 + 0,188 | 0 | 95 |
| | | 2,5 + 0,625 | 10 | 98 |
| E | | 1,0 | 30 | 95 |
| E | $A_3$ | 1,0 + 0,125 | 0 | 100 |

Tabelle 6 — Fortsetzung

| Herbizide Wirkstoffe | Antagonistisch wirkende Verbindung | Aufwandmenge (kg a.S./ha) | Testpflanzen und Schädigung (%) Kulturpflanze Zea mays | Testpflanzen und Schädigung (%) unerwünschte Pflanze Echinochloa crus-galli |
|---|---|---|---|---|
| F | | 1,0 | 60 | 98 |
| F | $A_3$ | 1,0 + 0,125 | 10 | 100 |
| G | | 1,5 | 15 | 98 |
| | | 2,5 | 30 | 100 |
| G | $A_3$ | 1,5 + 0,188 | 0 | 95 |
| | | 2,5 + 0,313 | 0 | 95 |
| H | | 1,5 | 80 | 98 |
| H | $A_3$ | 1,5 + 0,188 | 10 | 100 |
| I | | 2,0 | 25 | 100 |
| I | $A_3$ | 2,0 + 0,25 | 0 | 100 |
| K | | 1,0 | 40 | 98 |
| K | $A_3$ | 1,0 + 0,125 | 0 | 98 |
| K | $A_9$ | 1,0 + 0,125 | 25 | 100 |
| | | 1,0 + 0,25 | 10 | 100 |

0 = normaler Aufgang, keine Schädigung
100 = Pflanzen nicht aufgelaufen oder abgestorben

Tabelle 7 — Reduktion extrem starker Schäden an Mais durch 2-Chlor-2',6'-dimethyl-N-(pyrazolyl-1-yl-methyl)-acetanilid bei Zugabe antagonistisch wirkender Dichloracetamide bei Vorauflaufanwendung im Gewächshaus

| Herbizide Wirkstoffe | Antagonistisch wirkende Verbindung | Aufwandmenge (kg a.S./ha) | Testpflanzen und Schädigung (%) Kulturpflanze Zea mays | unerwünschte Pflanze Echinochloa crus-galli |
|---|---|---|---|---|
| A | - | 1,0 | 70 | 100 |
| A | $A_4$ | 1,0 + 0,125 | 25 | 100 |
| A | $A_5$ | 1,0 + 0,25 | 15 | 100 |
| A | $A_6$ | 1,0 + 0,125 | 10 | 100 |
| A | $A_7$ | 1,0 + 0,125 | 10 | 100 |

0 = keine Schädigung
100 = Pflanzen nicht aufgelaufen oder abgestorben

**Ansprüche**

1. Dichloracetamide der Formel (I)

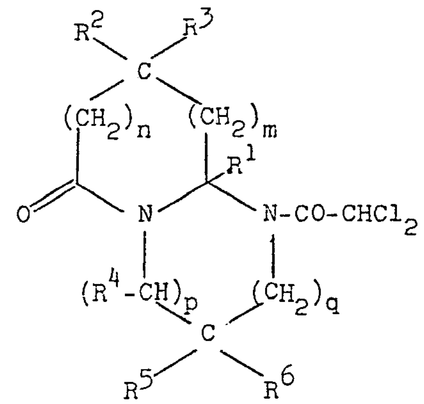

(I)

in der
$R^1$ für Wasserstoff, Methyl oder Ethyl,
$R^2$ und $R^3$ gleich oder verschieden sind und für Wasserstoff, Methyl oder Methoxy,
$R^4$ für Wasserstoff oder Methyl und
$R^5$ und $R^6$ gleich oder verschieden sind und für Wasserstoff oder Methyl stehen und
m 0 oder 1, n 1 oder 2, p 0, 1 oder 2 und q 0, 1 oder 2 bedeuten.

2. 5-Dichloracetyl-6-methyl-9-oxo-1,5-diazabicyclo[4.3.0]nonan.

3. 5-Dichloracetyl-3,3,6-trimethyl-9-oxo-1,5-diazabicyclo[4.3.0]nonan.

4. 4-Dichloracetyl-5-methyl-8-oxo-1,4-diazabicyclo[3.3.0]octan.

5. Verfahren zur Herstellung von Dichloracetamiden der Formel I gemäß Anspruch 1, dadurch gekennzeichnet, daß man Diazabicycloalkane der Formel III

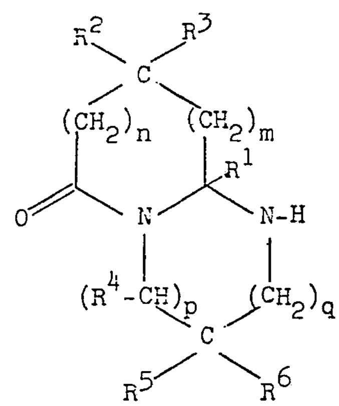

(III)

in der $R^1$, $R^2$, $R^3$, $R^4$, $R^5$, $R^6$ m, n, p und q die im Anspruch 1 angegebenen Bedeutungen haben, mit Dichloracetylchlorid in Gegenwart eines chlorwasserstoffbindenden Mittels in einem Lösungs- oder Verdünnungsmittel umsetzt.

6. Herbizide Mittel, gekennzeichnet durch einen Gehalt an mindestens einem Acetanilid der Formel II

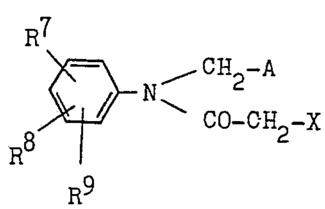

(II)

in der
$R^7$ Wasserstoff, einen unverzweigten oder verzweigten Alkyl- oder Alkoxyrest mit bis zu 5 Kohlenstoffatomen,
$R^8$ Wasserstoff, Halogen, einen unverzweigten oder verzweigten Alkyl- oder Alkoxyrest mit bis zu 5 Kohlenstoffatomen,
$R^9$ Wasserstoff, Halogen, einen unverzweigten oder verzweigten Alkyl- oder Alkoxyrest mit bis zu 5 Kohlenstoffatomen,

$R^7$ zusammen mit $R^8$ eine orthoständig verknüpfte, gegebenenfalls durch unverzweigte oder verzweigte Alkylgruppen mit bis zu 4 Kohlenstoffatomen substituierte Alkylenketten mit bis zu 6 Kohlenstoffatomen,

X Chlor oder Brom und

A einen Alkoxy- oder Alkoxyalkylrest mit bis zu 4 Kohlenstoffatomen oder ein über ein Ringstickstoffatom gebundenes Azol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy oder Alkoxycarbonyl mit bis zu 4 Kohlenstoffatomen in der Alkoxygruppe substituiert sein kann, wobei A auch für Salze der 2 oder 3 Stickstoffatome enthaltenden Azole stehen kann, bedeutet, als herbizidem Wirkstoff und mindestens einem Dichloracetamid der Formel I

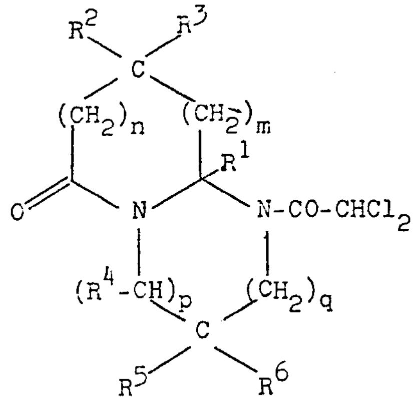

(I)

in der

$R^1$ für Wasserstoff, Methyl oder Ethyl,

$R^2$ und $R^3$ gleich oder verschieden sind und für Wasserstoff, Methyl oder Methoxy,

$R^4$ für Wasserstoff oder Methyl und

$R^5$ und $R^6$ gleich oder verschieden sind und für Wasserstoff oder Methyl stehen und

m 0 oder 1 ; n 1 oder 2, p 0, 1 oder 2 und q 0, 1 oder 2 bedeuten, als antagonistischem Mittel.

7. Herbizides Mittel nach Anspruch 6, dadurch gekennzeichnet, daß es als herbiziden Wirkstoff 2-Chlor-2',6'-dimethyl-N-(pyrazol-1-yl-methyl)-acetanilid enthält.

8. Herbizides Mittel nach Anspruch 6, dadurch gekennzeichnet, daß es als antagonistisches Mittel 5-Dichloracetyl-6-methyl-9-oxo-1,5-diazabicyclo[4.3.0]nonan enthält.

9. Herbizides Mittel nach Anspruch 6, dadurch gekennzeichnet, daß es als antagonistisches Mittel 5-Dichloracetyl-3,3,6-trimethyl-9-oxo-1,5-diazabicyclo[4.3.0]nonan enthält.

10. Herbizides Mittel nach Anspruch 6, dadurch gekennzeichnet, daß es als antagonistisches Mittel 4-Dichloracetyl-5-methyl-8-oxo-1,4-diazabicyclo[3.3.0]octan enthält.

11. Herbizides Mittel nach Anspruch 6, dadurch gekennzeichnet, daß das Anteilsverhältnis Acetanilid : Dichloracetamid bei gemeinsamer oder getrennter Ausbringung 1 : 2 bis 1 : 0,01 Gewichtsteile beträgt.

12. Verfahren zur selektiven Bekämpfung von unerwünschtem Pflanzenwuchs, dadurch gekennzeichnet, daß man Acetanilide der Formel II nach Anspruch 6 und Dichloracetamide der Formel I nach Anspruch 1 vor, bei oder nach der Aussaat der Kulturpflanzen, vor oder während des Auflaufens der Kulturpflanzen gleichzeitig oder nacheinander in beliebiger Reihenfolge ausbringt.

13. Verfahren zur selektiven Bekämpfung von unerwünschtem Pflanzenwuchs mit Acetaniliden der Formel II nach Anspruch 6, dadurch gekennzeichnet, daß man das Saatgut der Kulturpflanzen mit einem Dichloracetamid der Formel I nach Anspruch 1 behandelt.

14. Verfahren nach Anspruch 12 und 13, dadurch gekennzeichnet, daß die Kulturpflanzen Mais oder Cerealien sind.

## Claims

1. A dichloroacetamide of the formula (I)

(See the scheme, page 19)

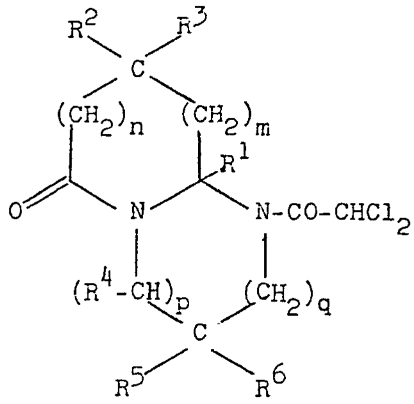

(I)

where

$R^1$ is hydrogen, methyl or ethyl,

$R^2$ and $R^3$ are identical or different and are hydrogen, methyl or methoxy,

$R^4$ is hydrogen or methyl, and

$R^5$ and $R^6$ are identical or different and are hydrogen or methyl, and

m is 0 or 1, n is 1 or 2, p is 0, 1 or 2 and q is 0, 1 or 2.

2. 5-Dichloroacetyl-6-methyl-9-oxo-1,5-diazabicyclo[4.3.0]nonane.

3. 5-Dichloroacetyl-3,3,6-trimethyl-9-oxo-1,5-diazabicyclo[4.3.0]nonane.

4. 4-Dichloroacetyl-5-methyl-8-oxo-1,4-diazabicyclo[3.3.0]octane.

5. A process for the preparation of a dichloroacetamide of the formula I as claimed in claim 1, wherein a diazabicycloalkane of the formula III

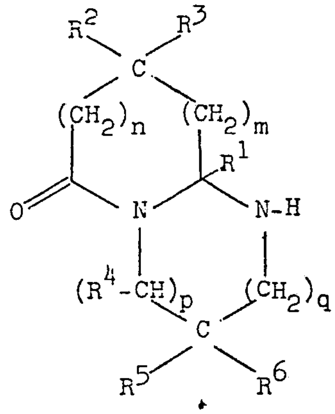

(III)

where $R^1$, $R^2$, $R^3$, $R^4$, $R^5$, $R^6$, m, n, p and q have the meanings given in claim 1, is reacted with dichloroacetyl chloride in the presence of a hydrogen chloride acceptor in a solvent or diluent.

6. A herbicidal agent containing, as herbicidal active ingredient, at least one acetanilide of the formula

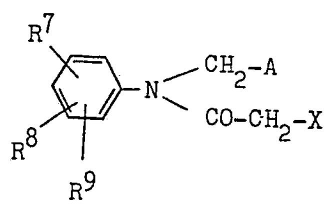

(II)

where

$R^7$ is hydrogen or linear or branched alkyl or alkoxy of up to 5 carbon atoms,

$R^8$ is hydrogen, halogen or linear or branched alkyl or alkoxy of up to 5 carbon atoms,

$R^9$ is hydrogen, halogen or linear or branched alkyl or alkoxy of up to 5 carbon atoms,

$R^7$ together with $R^8$ may also be alkylene of up to 6 carbon atoms which is ortho-linked to the benzene ring and is unsubstituted or substituted by linear or branched alkyl of up to 4 carbon atoms,

X is chlorine or bromine, and

A is alkoxy or alkoxyalkyl of up to 4 carbon atoms, or is an azole radical which is bonded via a ring nitrogen and which may be monosubstituted or polysubstituted by halogen, phenyl, alkyl, alkoxy, alkylthio or perfluoroalkyl, each of up to 4 carbon atoms, cyano, carboxyl or alkoxycarbonyl, where alkoxy is of up to 4 carbon atoms, and may also be a salified azole radical if the azole ring contains 2 or 3 nitrogens, and as antagonist, at least one dichloroacetamide of the formula

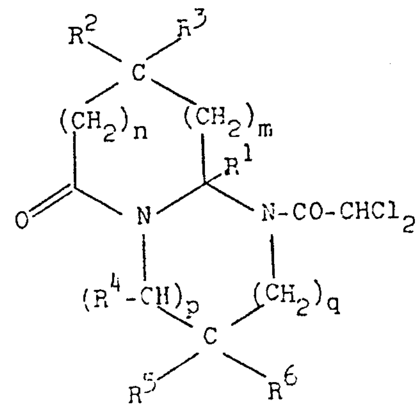

(I)

where

$R^1$ is hydrogen, methyl or ethyl,

$R^2$ and $R^3$ are identical or different and are hydrogen, methyl or methoxy,

$R^4$ is hydrogen or methyl, and

$R^5$ and $R^6$ are identical or different and are hydrogen or methyl, and

m is 0 or 1, n is 1 or 2, p is 0, 1 or 2 and q is 0, 1 or 2.

7. A herbicidal agent as claimed in claim 6, wherein the herbicidal active ingredient is 2-chloro-2',6'-dimethyl-N-(pyrazol-1-yl-methyl)-acetanilide.

8. A herbicidal agent as claimed in claim 6, wherein the antagonist is 5-dichloroacetyl-6-methyl-9-oxo-1,5-diazabicyclo[4.3.0]nonane.

9. A herbicidal agent as claimed in claim 6, wherein the antagonist is 5-dichloroacetyl-3,3,6-trimethyl-9-oxo-1,5-diazabicyclo[4.3.0]nonane.

10. A herbicidal agent as claimed in claim 6, wherein the antagonist is 4-dichloroacetyl-5-methyl-8-oxo-1,4-diazabicyclo[3.3.0]octane.

11. A herbicidal agent as claimed in claim 6, wherein the ratio of acetanilide to dichloroacetamide, applied conjointly or separately, is from 1 : 2 to 1 : 0.01 parts by weight.

12. A process for the selective control of unwanted plants, wherein the acetanilide of the formula II as claimed in claim 6 and the dichloroacetamide of the formula I as claimed in claim 1 are applied, simultaneously or one after the other in any sequence, before, during or after sowing of the crop plants, or before or during emergence of the crop plants.

13. A process for the selective control of unwanted plants with acetanilides of the formula II as claimed in claim 6, wherein the crop plant seed is treated with a dichloroacetamide of the formula I as claimed in claim 1.

14. A process as claimed in claims 12 and 13, wherein the crop plant is Indian corn or a cereal.

**Revendications**

1. Dichloracétamides de formule (I)

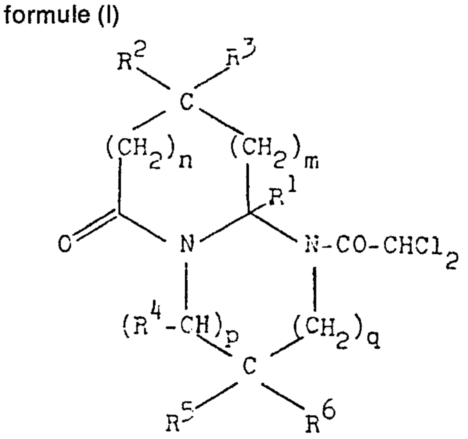

(I)

dans laquelle

$R^1$ représente hydrogène, méthyle ou éthyle,

$R^2$ et $R^3$ sont identiques ou différents et représentent hydrogène, méthyle ou méthoxy,

$R^4$ représente hydrogène ou méthyle et

$R^5$ et $R^6$ sont identiques ou différents et représentent hydrogène ou méthyle et

m représente 0 ou 1, n, 1 ou 2, p, 0, 1 ou 2 et q, 0, 1 ou 2.

2. 5-dichloracétyl-6-méthyl-9-oxo-1,5-diazabicyclo[4.3.0]nonane.
3. 5-dichloracétyl-3,3,6-triméthyl-9-oxo-1,5-diazabicyclo[4.3.0]nonane.
4. 4-dichloracétyl-5-méthyl-8-oxo-1,4-diazabicyclo[3.3.0]octane.
5. Procédé de préparation de dichloracétamides de formule 1 selon la revendication 1, caractérisé par le fait qu'on fait réagir avec du chlorure de dichloracétyle, en présence d'un agent liant l'acide chlorhydrique, dans un solvant ou diluant, des diazabicycloalcanes de formule III

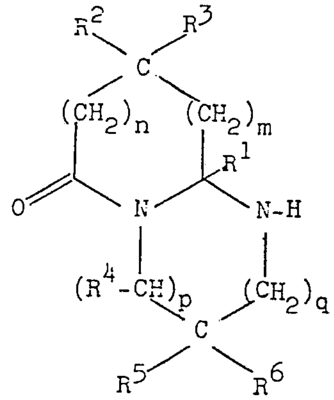

(III)

dans laquelle $R^1$, $R^2$, $R^3$, $R^4$, $R^5$, $R^6$, m, n, p et q ont les significations données dans la revendication 1.

6. Herbicide, caractérisé par le fait qu'il contient, comme principe actif herbicide, au moins un acétanilide de formule II

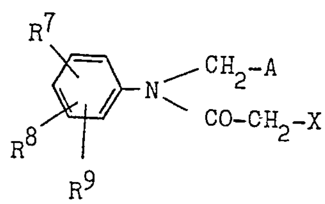

(II)

dans laquelle

$R^7$ représente, hydrogène, un reste alkyle ou alcoxy, non ramifié ou ramifié, ayant jusqu'à 5 atomes de carbone,

$R^8$ représente, hydrogène, halogène, un reste alkyle ou alcoxy, non ramifié ou ramifié, ayant jusqu'à 5 atomes de carbone,

$R^9$ représente, hydrogène, halogène, un reste alkyle ou alcoxy, non ramifié ou ramifié, ayant jusqu'à 5 atomes de carbone,

$R^7$ avec $R^8$ une chaîne alkylène ayant jusqu'à 6 atomes de carbone, liée en position ortho, éventuellement substituée par des groupes alkyle, ramifiés ou non, ayant jusqu'à 4 atomes de carbone,

X du chlore ou du brome et

A un reste alcoxy ou alcoxyalkyle ayant jusqu'à 4 atomes de carbone ou un azole lié par l'intermédiaire d'un atome d'azote du noyau, qui peut être substitué une fois ou plusieurs fois par halogène, phényle, des restes alkyl-, alcoxy-, alkylthio- ou perfluoralkyle ayant chacun jusqu'à 4 atomes de carbone cyano, carboxy ou alcoxy carbonyle ayant jusqu'à 4 atomes de carbone dans le groupe alcoxy, A pouvant aussi représenter des sels des azoles contenant 2 ou 3 atomes d'azote, et, comme agent antagonistique, au moins un dichloracétamide de formule I

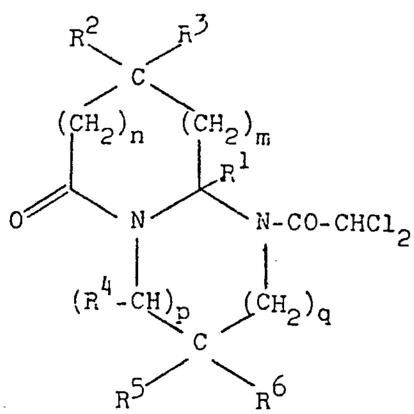

(I)

dans laquelle

$R^1$ représente hydrogène, méthyle ou éthyle,

$R^2$ et $R^3$ sont identiques ou différents et représentent hydrogène, méthyle ou méthoxy,

$R^4$ représente hydrogène ou méthyle et

$R^5$ et $R^6$ sont identiques ou différents et représentent hydrogène ou méthyle et

m représente 0, ou 1, n, 1 ou 2, p, 0, 1 ou 2 et q, 0, 1 ou 2.

7. Herbicide selon la revendication 6, caractérisé par le fait qu'il contient, comme principe actif herbicide, du 2-chloro-2',6'-diméthyl-N-(pyrazol-1-yl-méthyl)-acétanilide.

8. Herbicide selon la revendication 6, caractérisé par le fait qu'il contient comme agent antagonistique, du 5-dichloracétyl-6-méthyl-9-oxo-1,5-diazabicyclo[4.3.0]nonane.

9. Herbicide selon la revendication 6, caractérisé par le fait qu'il contient, comme agent antagonistique du 5-dichloracétyl-3,3,6-triméthyl-9-oxo-1,5-diazabicyclo[4.3.0]nonane.

10. Herbicide selon la revendication 6, caractérisé par le fait qu'il contient, comme agent antagonistique, du 4-dichloracétyl-5-méthyl-8-oxo-1,4-diazabicyclo[3.3.0]octane.

11. Herbicide selon la revendication 6, caractérisé par le fait que le rapport entre les parties en poids d'acétanilide et le dichloracétamide, pour une application simultanée ou distincte, est de 1/2 à 1/0,01.

12. Procédé de lutte sélective contre la croissance de plantes indésirables, caractérisé par le fait qu'on applique des acétanilides de formule II selon la revendication 6 et des dichloracétamides de formule I selon la revendication 1, avant, lors de, ou après l'ensemencement des plantes cultivées, avant ou pendant la levée des plantes cultivées, simultanément ou successivement dans un ordre arbitraire.

13. Procédé de lutte sélective contre la croissance de plantes indésirables, avec des acétanilides de formule II selon la revendication 6, caractérisé par le fait qu'on traite les semences des plantes cultivées avec un dichloracétamide de formule I selon la revendication 1.

14. Procédé selon les revendications 12 et 13, caractérisé par le fait que les plantes cultivées sont du maïs ou des céréales.